(19) Europäisches Patentamt / European Patent Office / Office européen des brevets

(11) **EP 3 878 352 B1**

(12) **EUROPEAN PATENT SPECIFICATION**

(45) Date of publication and mention of the grant of the patent:
**12.03.2025 Bulletin 2025/11**

(21) Application number: **20199622.0**

(22) Date of filing: **01.10.2020**

(51) International Patent Classification (IPC):
$A61B\ 5/00^{(2006.01)}$        $A61B\ 5/024^{(2006.01)}$
$A61B\ 5/0295^{(2006.01)}$     $A61B\ 5/029^{(2006.01)}$
$A61B\ 5/026^{(2006.01)}$       $G16H\ 50/20^{(2018.01)}$
$G16H\ 40/63^{(2018.01)}$       $G16H\ 50/30^{(2018.01)}$
$A61B\ 5/0285^{(2006.01)}$

(52) Cooperative Patent Classification (CPC):
**A61B 5/7239; A61B 5/02405; A61B 5/0261;
A61B 5/029; A61B 5/0295; G16H 40/63;
G16H 50/20; G16H 50/30;** A61B 5/0285;
A61B 5/6803; A61B 5/681; A61B 5/6898

(54) **APPARATUS AND METHOD FOR ESTIMATING BIO-INFORMATION**

VORRICHTUNG UND VERFAHREN ZUR SCHÄTZUNG VON BIOINFORMATIONEN

APPAREIL ET PROCÉDÉ D'ESTIMATION D'INFORMATIONS BIOLOGIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.03.2020 KR 20200029652**

(43) Date of publication of application:
**15.09.2021 Bulletin 2021/37**

(73) Proprietor: **Samsung Electronics Co., Ltd.
Gyeonggi-do 16677 (KR)**

(72) Inventors:
• **PARK, Chang Soon
  Chungju-si, Chungcheongbuk-do (KR)**
• **KWON, Ui Kun
  Hwaseong-si, Gyeonggi-do (KR)**
• **KIM, Young Soo
  Seoul (KR)**
• **YOON, Seung Keun
  Seoul (KR)**
• **JANG, Dae Geun
  Yongin-si, Gyeonggi-do (KR)**

(74) Representative: **Grünecker Patent- und
Rechtsanwälte
PartG mbB
Leopoldstraße 4
80802 München (DE)**

(56) References cited:
US-A1- 2017 209 055     US-A1- 2019 110 757
US-A1- 2020 054 223

• MOHAMED ELGENDI: "On the Analysis of
Fingertip Photoplethysmogram Signals",
CURRENT CARDIOLOGY REVIEWS, vol. 8, no. 1,
1 June 2012 (2012-06-01), pages 14 - 25,
XP055206723, ISSN: 1573-403X, DOI: 10.2174/
157340312801215782

## Description

BACKGROUND

### 1. Field

Example embodiments relate to an apparatus and method for non-invasively estimating bio-information.

### 2. Description of Related Art

With an aging population, soaring medical costs, and a lack of medical personnel for specialized medical services, research is being actively conducted on Information Technology (IT)-medical convergence technologies, in which IT and medical technology are combined. Particularly, monitoring the health condition of the human body is not limited to medical institutions, but is expanding to mobile healthcare fields that may monitor a user's health condition anywhere and anytime in daily life at home or the office. Typical examples of bio-signals, which indicate the health condition of individuals, include an electrocardiography (ECG) signal, a photoplethysmogram (PPG) signal, an electromyography (EMG) signal, and the like, and various bio-signal sensors have been developed to measure these signals in daily life. Particularly, a PPG sensor may estimate blood pressure of a human body by analyzing a shape of pulse waves which reflect cardiovascular status and the like.

According to studies on the PPG signal, the entire PPG signal is a superposition of a propagation wave moving from the heart toward the distal portions of the body, and reflection waves returning from the distal portions. Further, it has been known that information for estimating blood pressure may be obtained by extracting various features associated with the propagation wave or the reflection waves.

US 2020/0054223 A1 refers to an apparatus and method for estimating blood pressure. The apparatus for estimating blood pressure includes a bio-signal measurer configured to measure a bio-signal from a user and a processor configured to estimate blood pressure using the measured bio-signal. The processor extracts a first feature and a second feature from the bio-signal at an extraction time, estimates changes in the first feature and the second feature which have occurred during a time period from a calibration time at which the first feature and the second feature are calibrated to the extraction time at which the first feature and the second feature are extracted. The processor then estimates a blood pressure based on the changes in the first feature and the second feature.

US 2019/110757 A1 refers to a blood pressure estimating apparatus and blood pressure estimating method. The blood pressure estimating apparatus includes a sensor configured to obtain a bio-signal of an object, and a processor configured to extract a first cardiovascular feature and a second cardiovascular feature based on the bio-signal. Blood pressure is estimated based on a first changing tendency of the first cardiovascular feature from a first reference level and a second changing tendency of the second cardiovascular feature from a second reference level. The first changing tendency and the second changing tendency being independent from each other.

Summary

It is the object of the present invention to provide an improved method and apparatus for estimating bio-information.

This object is solved by the subject matter of the independent claims.

Preferred embodiments are defined by the dependent claims.

In accordance with an aspect of the disclosure, an apparatus for estimating bio-information includes a sensor configured to obtain a bio-signal of a subject; and a processor configured to: estimate a variation in a Cardiac Output (CO)-related feature from the bio-signal, and obtain a progressive wave component, which is associated with a Total Peripheral Resistance (TPR)-related feature, from the bio-signal based on an estimation result of estimating the variation.

The sensor may include a pulse wave sensor, and the pulse wave sensor may include a light source configured to emit light onto the subject, and a detector configured to detect light reflected or scattered from the subject.

The processor may obtain a heart rate from the bio-signal, and estimate the variation in the CO-related feature based on a variation in the heart rate compared to a reference heart rate obtained at a reference time.

The processor may, in response to the variation in the heart rate exceeding a predetermined threshold value: estimate that the variation in the CO-related feature is large, and obtain a progressive wave component, having a relatively large variation compared to a progressive wave component obtained at the reference time, from the bio-signal.

The processor may obtain a second-order differential signal of the bio-signal, and obtain the progressive wave component based on a first local minimum point of the second-order differential signal.

The processor may obtain, as the progressive wave component, one of a first amplitude of the bio-signal, which corresponds to a time of the first local minimum point, and a second amplitude of the bio-signal, which corresponds to an internal dividing point between the time of the first local minimum point and a time of a second local maximum point of the second-order differential signal.

The processor may detect an inflection point in a detection period of the second-order differential signal, and upon detecting the inflection point, obtain the progressive wave component based on the inflection point.

The detection period may include a time interval between a first local maximum point and the first local minimum point of the second-order differential signal.

The processor may, based on the variation in the heart rate being less than or equal to the predetermined threshold value, estimate that the variation in the CO-related feature is small, and obtain a progressive wave component, having a relatively small variation compared to the reference time, from the bio-signal.

The processor may obtain the progressive wave component based on a maximum amplitude point in a systolic phase of the bio-signal.

The processor may obtain, as the progressive wave component, one of a first amplitude of the maximum amplitude point, and a second amplitude of the bio-signal which corresponds to an internal dividing point between a time of a first local minimum point of a second-order differential signal of the bio-signal and a time of the maximum amplitude point.

The processor may estimate the variation in the CO-related feature based on a shape of a waveform of the bio-signal obtained at an estimation time.

The processor may, based on the waveform of the bio-signal rising from a systolic phase to a diastolic phase, estimate that the variation in the CO-related feature is large, and based on the waveform of the bio-signal rising from the diastolic phase to the systolic phase, estimate that the variation in the CO-related feature is small.

The processor may obtain the CO-related feature from the bio-signal, obtain the TPR-related feature based on the progressive wave component, and estimate bio-information based on the CO-related feature and the TPR-related feature.

The bio-information may include at least one of a blood pressure, a vascular age, an arterial stiffness, an aortic pressure waveform, a stress index, and a fatigue level.

In accordance with another aspect of the disclosure, a computer-implemented method of estimating bio-information, the method includes obtaining a bio-signal of a subject; estimating a variation in a Cardiac Output (CO)-related feature from the bio-signal; and obtaining a progressive wave component, which is associated with a Total Peripheral Resistance (TPR)-related feature, from the bio-signal based on an estimation result of the estimating the variation.

The estimating the variation in the CO-related feature may include obtaining a heart rate from the bio-signal, and estimating the variation in the CO-related feature based on a variation in the obtained heart rate compared to a reference heart rate obtained at a reference time.

The estimating the variation in the CO-related feature may include, based on the variation in the heart rate exceeding a predetermined threshold value, estimating that the variation in the CO-related feature is large, and the obtaining the progressive wave component comprises obtaining a progressive wave component, having a relatively large variation compared to a progressive wave component obtained at the reference time, from the bio-signal.

The obtaining the progressive wave component may include obtaining a second-order differential signal of the bio-signal; and obtaining the progressive wave component based on a first local minimum point of the obtained second-order differential signal.

The obtaining the progressive wave component may include obtaining, as the progressive wave component, one of a first amplitude of the bio-signal, which corresponds to a time of the first local minimum point, and a second amplitude of the bio-signal, which corresponds to an internal dividing point between the time of the first local minimum point and a time of a second local maximum point of the second-order differential signal.

The obtaining the progressive wave component may include detecting an inflection point in a detection period of the second-order differential signal; and upon detecting the inflection point, obtaining the progressive wave component based on the inflection point.

The estimating the variation in the CO-related feature may include, based on the variation in the heart rate being less than or equal to a predetermined threshold value, estimating that the variation in the CO-related feature is small, and the obtaining the progressive wave component comprises obtaining a progressive wave component, having a relatively small variation compared to the reference time, from the bio-signal.

The obtaining the progressive wave component may include detecting a maximum amplitude point in a systolic phase of the bio-signal; and obtaining the progressive wave component based on the maximum amplitude point.

The obtaining the progressive wave component may include obtaining, as the progressive wave component, one of a first amplitude of the maximum amplitude point, and a second amplitude of the bio-signal which corresponds to an internal dividing point between a time of a first local minimum point of a second-order differential signal of the bio-signal and a time of the maximum amplitude point.

The estimating the variation in the CO-related feature may include estimating the variation in the CO-related feature based on a shape of a waveform of the bio-signal obtained at an estimation time.

The estimating the variation in the CO-related feature may include based on the waveform of the bio-signal, which is obtained at the estimation time, rising from a systolic phase to a diastolic phase, estimating that the variation in the CO-related feature is large; and based on the waveform of the bio-signal, which is obtained at the estimation time, rising from the diastolic phase to the systolic phase, estimating that the variation in the CO-related feature is small.

The method of estimating bio-information may include obtaining the CO-related feature from the bio-signal; obtaining the TPR-related feature based on the obtained progressive wave component; and estimating bio-information based on the CO-related feature and the TPR-related feature.

## BRIEF DESCRIPTION OF THE DRAWINGS

The above and other aspects, features, and advantages of certain example embodiments of the present disclosure will become more apparent the following description taken in conjunction with the accompanying drawings, in which

FIG. 1 is a block diagram showing an apparatus for estimating bio-information according to an example embodiment.
FIG. 2 is a block diagram showing an apparatus for estimating bio-information according to another example embodiment.
FIG. 3 is a block diagram showing a processor according to the example embodiments of FIGS. 1 and 2.
FIGS. 4A to 4F are diagrams showing examples of extracting a progressive wave component from a bio-signal.
FIG. 5 is a flowchart showing a method of estimating bio-information according to an example embodiment.
FIG. 6 is a diagram showing a wearable device according to an example embodiment.
FIG. 7 is a diagram showing a smart device according to an example embodiment.

## DETAILED DESCRIPTION

Details of example embodiments are included in the following detailed description and drawings. Advantages and features of the present invention, and a method of achieving the same will be more clearly understood from the following example embodiments described in detail with reference to the accompanying drawings. Throughout the drawings and the detailed description, unless otherwise described, the same drawing reference numerals will be understood to refer to the same elements, features, and structures. The relative size and depiction of these elements are not necessarily to scale and may be exaggerated for clarity, illustration, and convenience.

Although the terms first, second, etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as 'part' or 'module', etc., should be understood as a unit for performing at least one function or operation and that may be embodied as hardware, software, or a combination thereof. As used herein, expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, or all of a, b, and c.

Hereinafter, example embodiments of an apparatus and method for estimating bio-information will be described in detail with reference to the accompanying drawings.

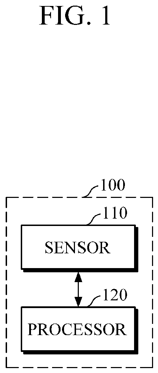

FIG. 1 is a block diagram showing an apparatus for estimating bio-information according to an example embodiment. The apparatus 100 for estimating bio-information may be embedded in a terminal, such as a smartphone, a tablet personal computer (PC), a desktop computer, a laptop computer, and the like, or may be manufactured as an independent hardware device. If the apparatus 100 for estimating bio-information is manufactured as an independent hardware device, the device may be a wearable device worn by a user so that a user may easily measure bio-information while wearing the device. Examples of the wearable device may include a wristwatch-type wearable device, a bracelet-type wearable device, a wristband-type wearable device, a ring-type wearable device, a glasses-type wearable device, a headband-type wearable device, or the like, but the wearable device is not limited thereto, and may be modified for various purposes, such as a fixed type device and the like used in medical institutions for measuring and analyzing bio-information.

Referring to FIG. 1, the apparatus 100 for estimating bio-information includes a sensor 110 and a processor 120.

As shown in FIG. 1, the sensor 110 may obtain a bio-signal from an object, and may transmit the obtained bio-signal to the processor 120. The bio-signal may include a photoplethysmogram (PPG) signal (also referred to as a "pulse wave signal"). However, the bio-signal is not limited thereto, and may include various bio-signals, such as an electrocardiography (ECG) signal, a photoplethysmogram (PPG) signal, an electromyography (EMG) signal, and the like, which may be modeled by a sum of a plurality of waveform components.

For example, the sensor 110 may include a PPG sensor for measuring the PPG signal. The PPG sensor may include a light source for emitting light onto an object and a detector for measuring the PPG signal by detecting light emanating from the object when light, emitted onto the object by the light source, is scattered or reflected from body tissue of the object. In this case, the light source may include at least one of a light emitting diode (LED), a laser diode (LD), and a phosphor, but is not limited thereto. The detector may include a photo diode.

Upon receiving a control signal from the processor 120, the sensor 110 may direct the PPG sensor to obtain a pulse wave signal from the object. In this case, the object may be a body part which comes into contact with or is adjacent to the PPG sensor, and may be a body part where pulse waves may be easily measured using photoplethysmography. For example, the object may be an area on the wrist that is adjacent to the radial artery, and may include an upper portion of the wrist where veins or capillaries are located. In the case where the pulse waves are measured on an area of skin where the radial artery passes, measurement may be relatively less affected by external factors, such as the thickness of skin tissue in the wrist and the like, which may cause errors in measurement. However, the skin area is not limited thereto, and may be distal portions of the body, such as fingers, toes, and the like where blood vessels are densely located.

Upon receiving a request for estimating bio-information from a user, the processor 120 may generate a control signal for controlling the sensor 110, and may transmit the control signal to the sensor 110. Further, the processor 120 may receive a bio-signal from the sensor 110 and may estimate bio-information by analyzing the received bio-signal. In this case, bio-information may include blood pressure, vascular age, arterial stiffness, aortic pressure waveform, stress index, fatigue level, and the like, but is not limited thereto..

Upon receiving the bio-signal from the sensor 110, the processor 120 may perform preprocessing, such as filtering for removing noise, amplifying the bio-signal, converting the signal into a digital signal, and the like.

The processor 120 may extract features, required for estimating bio-information, by analyzing a waveform of the received bio-signal, and may estimate bio-information by using the extracted features. Bio-information may include blood pressure, vascular age, arterial stiffness, aortic pressure waveform, stress index, fatigue level, and the like, but is not limited thereto. For convenience of explanation, the following description will be made using blood pressure as an example of bio-information.

For example, it has been known that a variation of Mean Arterial Pressure (MAP) is proportional to cardiac output (CO) and total peripheral resistance (TPR), as represented by the following Equation (1).

$$\Delta MAP = CO \times TPR \qquad (1)$$

Herein, $\Delta$MAP denotes a difference in MAP between the left ventricle and the right atrium, in which MAP of the right atrium is generally in a range of 3 mmHg to 5 mmHg, such that the MAP in the right atrium is similar to MAP in the left ventricle or MAP of the upper arm. If absolute actual CO and TPR values are known, MAP may be obtained for the aorta or the upper arm. However, it may be difficult to estimate absolute CO and TPR values based on a bio-signal. The human body may regulate blood pressure under normal conditions. For example, when blood pressure appears to increase due to a sharp increase in cardiac output, a blood vessel diameter is relaxed such that the total peripheral resistance may be reduced, thereby allowing the blood pressure to return to a normal level.

The processor 120 may obtain a feature related to cardiac output (CO) (hereinafter referred to as a CO-related feature) and a feature related to total peripheral resistance (TPR) (hereinafter referred to as a TPR-related feature) from a pulse wave signal, and may estimate blood pressure by using the CO-related feature and the TPR-related feature. The CO-related feature may be a feature value which shows an increasing/decreasing trend in relation to an actual CO value which relatively increases/decreases when an actual TPR value does not change significantly compared to a resting state. Further, the TPR-related feature may be a feature value which shows an increasing/decreasing trend in relation to an actual TPR value which relatively increases/decreases when an actual CO value does not change significantly compared to a resting state.

According to the example embodiments of the present disclosure, the processor 120 may obtain the CO-related feature and the TPR-related feature adaptively from the bio-signal by using a complementary relationship between the CO and the TPR. For example, the processor 120 may estimate a variation in the CO-related feature at a time of the blood pressure estimation based on heart rate (HR) and stroke volume (SV) characteristics which constitute the cardiac output. Further, the processor 120 may adaptively obtain a progressive wave component, associated with the TPR-related feature, based on the estimated variation in the CO-related feature. Upon estimating that the variation in the CO-related feature is relatively large at the time of the blood pressure estimation compared to a time when calibration is performed while a user is at rest (hereinafter referred to as a "reference time"), the processor 120 may select a TPR-related feature value which also has a large variation. In contrast, upon estimating that the variation in the CO-related feature is relatively small, the processor 120 may select a TPR-related feature value which also has a small variation.

Upon obtaining the CO-related feature and the TPR-related feature, the processor 120 may estimate blood pressure by applying a pre-defined blood pressure estimation model. The blood pressure estimation model may be expressed in the form of a linear or nonlinear function which defines a correlation between the CO-related feature and the TPR-related feature and blood pressure.

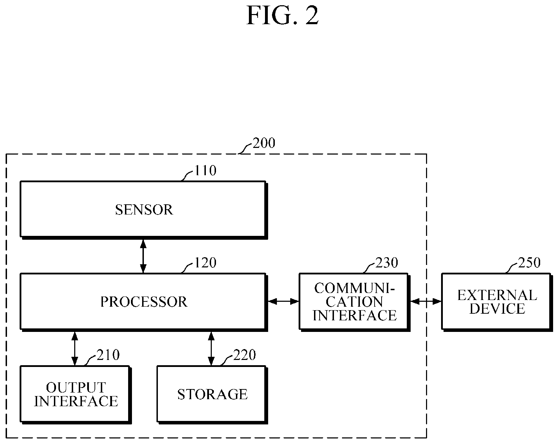

FIG. 2 is a block diagram showing an apparatus for estimating bio-information according to another example embodiment.

Referring to FIG. 2, the apparatus 200 for estimating bio-information includes a sensor 110, a processor 120, an output interface 210, a storage 220, and a communication interface 230.

The sensor 110 may measure a bio-signal from an object, and the processor 120 may estimate bio-information by using bio-signal information obtained by the sensor 110.

The output interface 210 may output bio-signal information, obtained by the sensor 110, and various processing results of the processor 120, and may provide the output information for a user. The output interface 210 may provide the information by various visual/non-visual methods using a display module, a speaker, a haptic device, and the like which are mounted in the apparatus 200 for estimating bio-information.

For example, once a user's blood pressure is estimated, the output interface 210 may output the estimated blood pressure by using various visual methods, such as by changing color, line thickness, font, and the like, based on whether the estimated blood pressure value falls within or outside a normal range. Alternatively, the output interface 210 may output the estimated blood pressure by voice, or may output the estimated blood pressure using non-visual methods by providing different vibrations or tactile sensations and the like corresponding to abnormal blood pressure levels. In addition, upon comparing the measured blood pressure with a previous measurement history, if it is determined that the measured blood pressure is abnormal, the output interface 210 may warn a user, or may provide information to guide a user's action such as food information that the user should be careful about, information for booking a hospital appointment, and the like.

The storage 220 may store reference information, bio-signals, obtained features, bio-information estimation results, and the like. The reference information may include user information, such as a user's age, sex, occupation, current health condition, and the like, and/or information on a bio-information estimation model, and the like, but the reference information is not limited thereto. The storage 220 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

Upon receiving a control signal, including access information of an external device 250, from the processor 120, the communication interface 230 may access a communication network using communication techniques to be connected to the external device 250. Upon connection with the external device 250, the communication interface 230 may receive a variety of information related to estimating bio-information from the external device 250, and may transmit the bio-signal information obtained by the sensor 110, the bio-information estimated by the processor 120, and the like to the external device 250. Examples of the external device 250 may include other apparatus for estimating bio-information, a cuff manometer for measuring cuff blood pressure and the like, a smartphone, a tablet PC, a desktop computer, a laptop computer, and the like, but the external device 250 is not limited thereto.

Examples of the communication techniques may include Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, and mobile communication. However, these are merely exemplary and is not intended to be limiting.

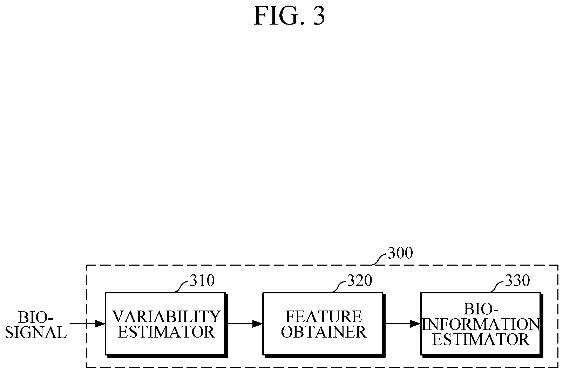

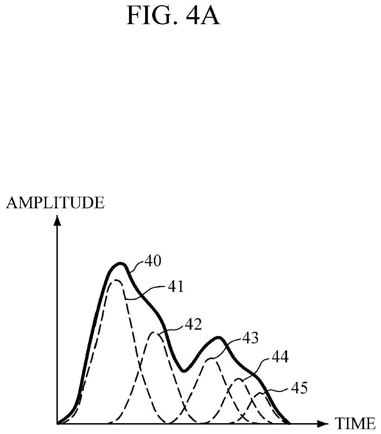

FIG. 3 is a block diagram showing a processor according to the example embodiments of FIGS. 1 and 2. FIGS. 4A through 4F are diagrams explaining examples of extracting a progressive wave component from a bio-signal.

Referring to FIG. 3, a processor 300 may include a variability estimator 310, a feature obtainer 320, and a bio-information estimator 330.

The variability estimator 310 may estimate variability in the CO-related feature by using a bio-signal acquired from an object.

For example, once a bio-signal is acquired from an object for estimating bio-information, the variability estimator 310 may obtain a heart rate, which is an element constituting cardiac output. The heart rate may be obtained by using one period of the bio-signal. Further, the variability estimator 310 may obtain a heart rate variation compared to a reference heart rate, and may estimate variability in CO-related feature based on the heart rate variation. In this case, the reference heart rate is a heart rate measured when a user is at rest, and may be obtained from a bio-signal obtained at a reference time or may be obtained by an external apparatus for measuring a heart rate.

Upon obtaining the heart rate variation, the variability estimator 310 may estimate variability in the CO-related feature by comparing the obtained heart rate variation with a predetermined threshold value. If the heart rate variation exceeds a predetermined threshold value, the variability estimator 310 may estimate that variability in the CO-related feature is high. In contrast, if the heart rate variation is less than or equal to the predetermined threshold value, the variability estimator 310 may estimate that variability in the CO-related feature is relatively low. The predetermined threshold value may be defined as a value personalized according to types of bio-information, a user's gender, age, health condition, vascular specificity, and the like, and may be adjusted by calibration. However, the threshold value is not limited thereto, and may be defined as a general fixed value which is obtained from a plurality of users.

In another example, the variability estimator 310 may predict variability in stroke volume (SV), which indicates cardiac output, by analyzing a shape of a waveform of a reference bio-signal measured at the reference time, and may estimate a variation in CO-related feature based on the prediction result.

For example, if a waveform of the reference bio-signal at the reference time rises from a systolic phase to a diastolic phase, the variability estimator 310 may predict that variability in stroke volume (SV) is high at the estimation time of a bio-signal, and may estimate that the variation in the CO-related feature is also large, which is extracted from the bio-signal at the estimation time of the bio-signal. In contrast, if a waveform of the reference bio-signal at the reference time rises from the diastolic phase to the systolic phase, the variability estimator 310 may predict that variability in stroke volume (SV) is low at the estimation time of the bio-signal, and may estimate that the variation in CO-related feature is also small. The systolic phase may refer to an interval from a start point of the bio-signal to a dicrotic notch point, and the diastolic phase may refer to a time interval after the dicrotic notch.

FIG. 4A is a diagram showing a waveform of a pulse wave signal 40 which is a superposition of five constituent pulses 41, 42, 43, 44 and 45. By using information associated with each of the constituent pulses 41, 42, 43, 44 and 45 of the pulse wave signal 40, features having a high correlation with blood pressure may be obtained. Generally, pulses up to the third constituent pulse are mainly used to estimate blood pressure. Pulses after the third pulse may not be observed depending on individuals in some cases, and are difficult to be found due to noise or have a low correlation with estimation of blood pressure.

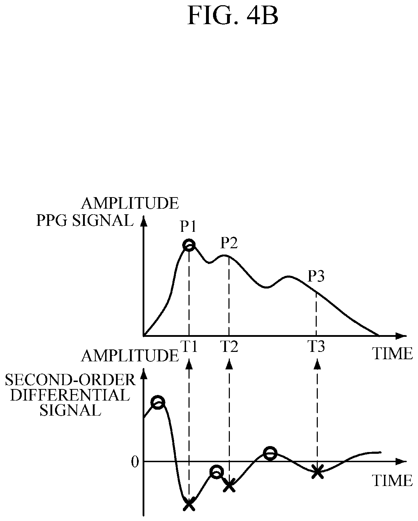

For example, referring to FIG. 4B, the variability estimator 310 may detect a first local minimum point T1, a second local minimum point T2 and a third local minimum point T3 from a second-order differential signal of the measured bio-signal. Further, the variability estimator 310 may extract a first amplitude P1, a second amplitude P2 and a third amplitude P3, which correspond to the first local minimum point T1, the second local minimum point T2 and the third local minimum point T3 respectively, from the measured bio-signal, and may analyze a shape of a waveform of the measured bio-signal by using the extracted amplitudes P1, P2 and P3. For example, if P2 is greater than a value obtained by multiplying P1 by a predetermined first value and P3 is greater than a value obtained by multiplying P2 by a predetermined second value, the variability estimator 310 may determine that the waveform of the reference bio-signal rises from the systolic phase to the diastolic phase; and if not, the variability estimator 310 may determine that the waveform of the measured bio-signal rises from the diastolic phase to the systolic phase.

The feature obtainer 320 may obtain the CO-related feature and the TPR-related feature from the measured bio-signal based on an estimation result of the variability estimator 310.

The feature obtainer 320 may obtain a heart rate (HR) from the measured bio-signal, and may set the obtained HR as the CO-related feature. However, the feature obtainer 320 is not limited thereto, and may obtain other additional information and combine the additional information to obtain the CO-related feature. For example, the additional information may include the waveform of the measured bio-signal, a time Tmax and/or an amplitude Pmax at a maximum point in the systolic phase of the bio-signal, a time Tmin and/or an amplitude Pmin at a minimum point of the bio-signal, a total or partial area PPGarea of the waveform of the bio-signal, a duration PPGdur of the bio-signal, amplitudes P1, P2 and P3 and/or times T1, T2 and T3 of the constituent pulse waveforms constituting the bio-signal, and the like; and for example, PPGarea, P3/Pmax, Pmax/PPGarea, 1/PPGdur and the like may be obtained as the CO-related feature.

Further, the feature obtainer 320 may obtain the TPR-related feature from the measured bio-signal based on the variation in the CO-related feature, which is estimated by the variability estimator 310. The TPR-related feature may be defined as, for example, a ratio P2/P1 between an amplitude P1 of a progressive wave component and an amplitude P2 of a first reflection wave component of the constituent pulse waveform constituting the measured bio-signal. However, the TPR-related feature is not limited thereto, and the feature obtainer 320 may obtain, for example, 1/(T3-T1), 1/(T3-Tmax), 1/(T2-T1), P3/Pmax, P3/P1, and the like, as the TPR-related feature by combining the above additional information.

Based on the estimated variation in the CO-related feature, the feature obtainer 320 may adaptively obtain an amplitude of the progressive wave component which is associated with the TPR-related feature. Once the variability estimator 310 estimates that the variation in the CO-related feature is large, the feature obtainer 320 may obtain, as an element of the TPR-related feature, a progressive wave component having a relatively large variation compared to the reference time.

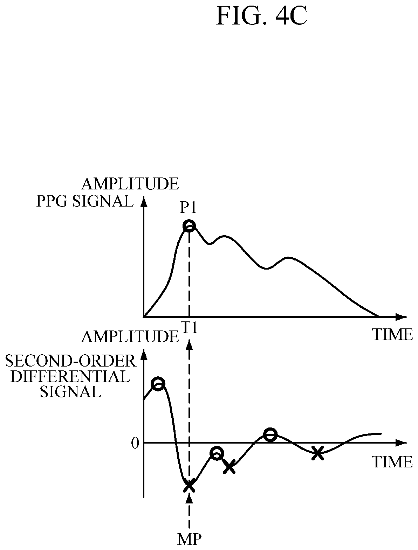

For example, referring to FIG. 4C, the feature obtainer 320 may obtain a second-order differential signal of the measured bio-signal, and may obtain the amplitude P1 of the bio-signal, which corresponds to the time T1 of the first local minimum point MP, as a progressive wave component having a large variation. However, the feature obtainer 320 is not limited thereto, and may also obtain an amplitude of the measured bio-signal, which corresponds to an internal dividing point between a time of the first local minimum point MP and a time of a second local maximum point of the second-order differential signal, as the progressive wave component. The internal dividing point may be obtained by applying a predetermined weight to each of the time of the first local minimum point and the time of the second local maximum point.

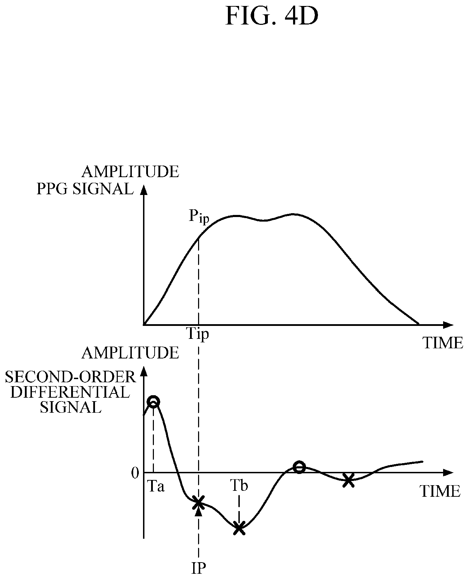

In yet another example, shown in FIG. 4D, the feature obtainer 320 may detect an inflection point IP in a detection period of the second-order differential signal. Upon detecting the inflection point IP, the feature obtainer 320 may obtain a progressive wave component, having a relatively large variation, based on the detected inflection point IP. For example, the feature obtainer 320 may obtain, as the progressive wave component, an amplitude Pip of the bio-signal, which corresponds to a time Tip of the inflection point IP. In this case, the inflection point may be a point, at which a waveform of the second-order differential signal changes from being convex "downward" to convex "upward" with time in the detection period of the second-order differential signal; and the detection period may include a time interval between a time of a first local maximum point and the time of the first local minimum point of the second-order differential signal.

In addition, once the variability estimator 310 estimates that the variation in the CO-related feature is relatively small, the feature obtainer 320 may obtain, as an element of the TPR-related feature, a progressive wave component having a relatively small variation compared to the reference time.

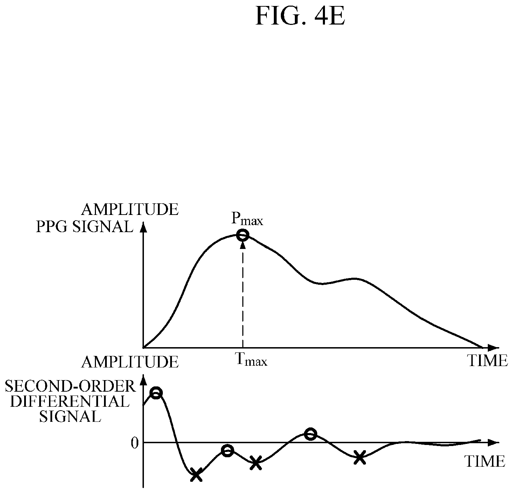

For example, referring to FIG. 4E, the feature obtainer 320 may obtain, as the progressive wave component having a small variation, an amplitude Pmax corresponding to a maximum amplitude point Tmax in the systolic phase of the measured bio-signal. The systolic phase may refer to an interval from a start point of the bio-signal to a dicrotic notch point, and the dicrotic notch point may refer to a time point of a third local maximum point of a second-order differential signal.

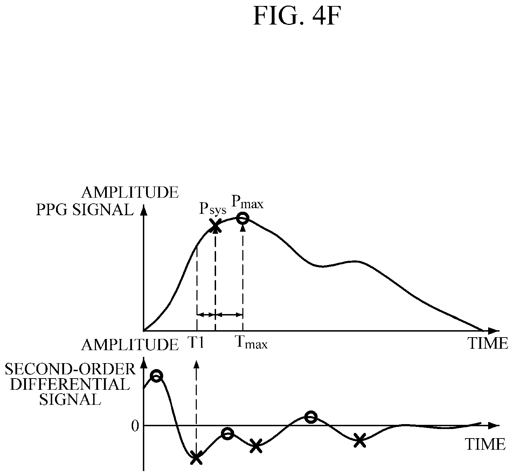

In another example, shown in FIG. 4F, the feature obtainer 320 may obtain a second-order differential signal, may detect a time T1 of the first local minimum point from the second-order differential signal and a maximum amplitude point Tmax in the systolic phase of the bio-signal, and may obtain an amplitude Psys of an internally dividing point between the time T1 of the first local minimum point and the maximum amplitude point Tmax as the progressive wave component having a small variation,.

Upon obtaining the progressive wave component based on the variation in the CO-related feature, the feature obtainer 320 may obtain the TPR-related feature based on the progressive wave component. For example, the feature obtainer 320 may obtain an amplitude of the bio-signal, which corresponds to a time of a second local minimum point of the second-order differential signal, as an amplitude component of a first reflection wave, and may obtain the TPR-related feature by obtaining a ratio between an amplitude of the progressive wave component and the amplitude component of the first reflection wave. However, the TPR-related feature is not limited thereto, and the feature obtainer 320 may obtain other additional information described above in addition to the first reflection wave component, and may also obtain the TPR-related feature by combining two or more of the progressive wave, the first reflection wave and other additional information.

Once the CO-related feature and the TPR-related feature are obtained from the measured bio-signal, the bio-information estimator 330 may estimate bio-information by applying a bio-information estimation model which defines a correlation between the CO-related feature, the TPR-related feature and bio-information.

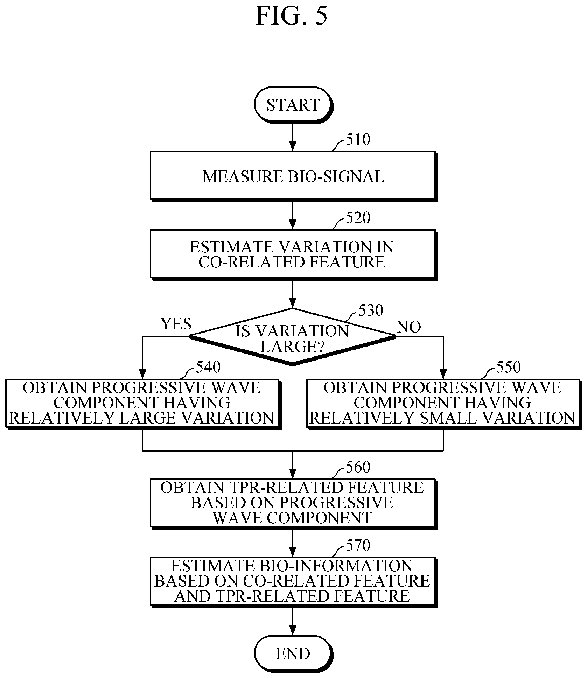

FIG. 5 is a flowchart showing a method of estimating bio-information according to an example embodiment.

The method of FIG. 5 is an example of a method of estimating bio-information, which is performed by the apparatuses 100 and 200, and will be briefly described below in order to avoid redundancy.

Upon receiving a request for estimating bio-information, the apparatuses 100 and 200 for estimating bio-information may measure a bio-signal from a user in operation 510.

The apparatuses 100 and 200 for estimating bio-information may provide an interface for various interactions with a user, and may receive the request for estimating bio-information from the user through the provided interface. Alternatively, the apparatuses 100 and 200 may receive a request for estimating bio-information from an external device. In this case, the request for estimating bio-information from the external device may include a request for providing a bio-information estimation result. In a case where the external device includes a bio-information estimation algorithm, the request for estimating bio-information may also include a request for providing feature information. The external device may be a smartphone, a tablet PC, and the like which may be carried by the user.

Then, the apparatuses 100 and 200 for estimating bio-information may estimate a variation in the CO-related feature in operation 520, and may estimate whether the estimated variation is large or small in operation 530.

For example, the apparatuses 100 and 200 may obtain a heart rate, which is an element of cardiac output, from the bio-signal obtained in 510. Further, the apparatuses 100 and 200 may detect a heart rate variation compared to a reference heart rate measured in a resting state at a reference time, and may estimate the variation in the CO-related feature based on the heart rate variation. For example, if the heart rate variation exceeds a predetermined threshold value, the apparatuses 100 and 200 may estimate that the variation in the CO-related feature is large, and if the heart rate variation is less than or equal to the predetermined threshold value, the apparatuses 100 and 200 may estimate that the variation in CO-related feature is small.

In another example, the apparatuses 100 and 200 may predict a variation in stroke volume, which is an element of cardiac output, based on a shape of a waveform of a reference bio-signal measured at the reference time, and may estimate the variation in the CO-related feature based on the prediction result. For example, if the waveform of the reference bio-signal rises from a systolic phase to a diastolic phase, the apparatuses 100 and 200 may predict that the variation in stroke volume at the estimation time of bio-information is large and may estimate that the variation in the CO-related feature is large; and in the opposite case, the apparatuses 100 and 200 may predict that the variation in stroke volume is small and may estimate that the variation in the CO-related feature is small.

Then, upon estimating that the variation in the CO-related feature is large, the apparatuses 100 and 200 for estimating bio-information may obtain, as an element of a TPR-related feature, a progressive wave component having a relatively large variation compared to the reference time in 540.

For example, the apparatuses 100 and 200 for estimating bio-information may obtain a second-order differential signal of the measured bio-signal, and may obtain an amplitude of the measured bio-signal, which corresponds to a time of a first local minimum point of the second-order differential signal, as a progressive wave component having a large variation. Alternatively, the apparatuses 100 and 200 may obtain an amplitude of the measured bio-signal, which corresponds to an internal dividing point between the time of the first local minimum point and a time of a second local maximum point, as the progressive wave component. In this case, the internal dividing point may be obtained by applying a predetermined weight to each of the time of the first local minimum point and the time of the second local maximum point. Alternatively, the apparatuses 100 and 200 may detect an inflection point in a detection period of the second-order differential signal, and upon detecting the inflection point, the apparatuses 100 and 200 may obtain an amplitude of the measured bio-signal, which corresponds to the detected inflection point, as a progressive wave component having a relatively large variation.

Subsequently, upon estimating that the variation in the CO-related feature is small, the apparatuses 100 and 200 may obtain a progressive wave component having a relatively small variation compared to the reference time in operation 550. For example, the apparatuses 100 and 200 may obtain an amplitude of the measured bio-signal, which corresponds to a maximum amplitude point in the systolic phase of the bio-signal, as the progressive wave component having a small variation. Alternatively, the apparatuses 100 and 200 may detect a first local minimum point from the second-order differential signal of the measured bio-signal, and may obtain an amplitude of an internal dividing point between the first local minimum point and the maximum amplitude point as the progressive wave component having a small variation.

Next, the apparatuses 100 and 200 may obtain the CO-related feature based on the bio-signal obtained in operation 510, and may obtain the TPR-related feature in operation 560 based on the progressive wave component obtained in operations 540 or 550.

Then, the apparatuses 100 and 200 may estimate bio-information based on the obtained CO-related feature and TPR-related feature in operation 570. Upon estimating bio-information, the apparatuses 100 and 200 may provide the estimation result for a user. In this case, the apparatuses 100 and 200 may provide the estimated bio-information for the user by various visual/non-visual methods. In addition, the apparatuses 100 and 200 may determine the user's health condition based on the estimated bio-information, and may provide a warning or a response action for the user based on the determination.

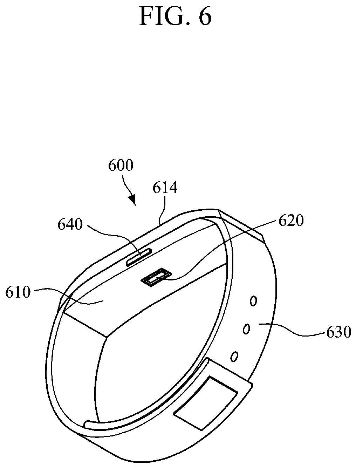

FIG. 6 is a diagram showing a wearable device according to an example embodiment. The aforementioned example embodiments of the apparatuses 100 and 200 may be mounted in a smart watch or a smart band-type wearable device, as shown in FIG. 6, which may be worn on a wrist, but are not limited thereto.

Referring to FIG. 6, the wearable device 600 may include a main body 610 and a strap 630.

The main body 610 may be formed to have various shapes, and may include modules which are mounted inside or outside of the main body 610 to perform the aforementioned function of estimating bio-information and various other functions. A battery may be embedded in the main body 610 or the strap 630 to supply power to various modules of the wearable device 600.

The strap 630 may be connected to the main body 610. The strap 630 may be flexible so as to be bent around a user's wrist. The strap 630 may be bent in a manner that allows the strap 630 to be detached from the user's wrist or may be formed as a band that is not detachable. Air may be injected into the strap 630 or an airbag may be included in the strap 630, so that the strap 630 may have elasticity according to a change in pressure applied to the wrist, and the strap 630 may transmit the change in pressure of the wrist to the main body 610.

The main body 610 may include a sensor 620 for measuring a bio-signal. The sensor 620 may be mounted on a rear surface of the main body 610, which may come into contact with the upper portion of a user's wrist. The sensor 620 may include a light source for emitting light onto the skin of the user's wrist and a detector for detecting light scattered or reflected from the tissue of the user's wrist. The sensor 620 may further include a contact pressure sensor for measuring contact pressure applied by the user's wrist.

A processor may be mounted in the main body 610. The processor may be electrically connected to various modules, mounted in the wearable device 600, to control operations thereof. Further, the processor may estimate bio-information by using bio-signals measured by the sensor 620.

For example, the processor may obtain a CO-related feature and a TPR-related feature, which are associated with blood pressure, from the bio-signal and may estimate blood pressure by using the obtained CO-related feature and TPR-related feature. When worn on a user's wrist, the wearable device 600 may measures a bio-signal from a capillary portion on an upper part of the wrist, thus acquiring a bio-signal mainly having low frequency components. Accordingly, in order to stably extract a progressive wave component associated with the TPR-related feature even when the bio-signal mainly having low-frequency components is acquired, the processor may estimate a variation in the CO-related feature based on a heart rate or a waveform shape of the measured bio-signal, and may obtain a progressive wave component adaptively based on the estimated variation in the CO-related feature.

In the case where the sensor 620 includes a contact pressure sensor, the processor may monitor a contact state of the user based on contact pressure between the wrist and the sensor 620, and may provide guide information on a contact position and/or a contact state for a user through a display.

Further, the main body 610 may include a memory which stores processing results of the processor and a variety of information. In this case, the variety of information may include reference information related to estimating bio-information, as well as information associated with functions of the wearable device 600.

In addition, the main body 610 may also include a manipulator 640 which receives a user's control command and transmits the received control command to the processor. The manipulator 640 may include a power button to input a command to turn on/off the wearable device 600.

A display 614 may be mounted on a front surface of the main body 610, and may include a touch panel for receiving a touch input. The display 614 may receive a touch input from a user, may transmit the received touch input to the processor, and may display a processing result of the processor. For example, the display 614 may display an estimated bio-information value and warning/alarm information.

A communication interface, provided for communication with an external device such as a user's mobile terminal, may be mounted in the main body 610. The communication interface may transmit a bio-information estimation result to an external device, e.g., a user's smartphone, to display the result to the user. However, the communication interface is not limited thereto, and may transmit and receive a variety of necessary information.

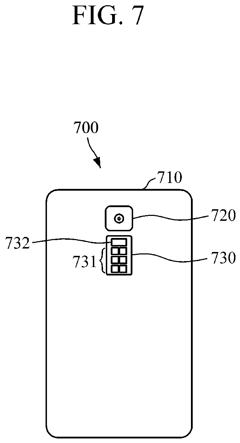

FIG. 7 is a diagram illustrating a smart device according to an example embodiment. The smart device may be a smartphone, a tablet PC, and the like, and may include the aforementioned apparatuses 100 and 200 for estimating bio-information.

Referring to FIG. 7, the smart device 700 may include a main body 710 and a sensor 730 mounted on one surface of the main body 710. The sensor 730 may include a pulse wave sensor including at least one or more light sources 731 and a detector 732. As shown in FIG. 7, the sensor 730 may be mounted on a rear surface of the main body 710, but is not limited thereto, and may be configured in combination with a fingerprint sensor or a touch panel mounted on a front surface of the main body 710.

In addition, a display may be mounted on a front surface of the main body 710. The display may visually display a bio-information estimation result and the like. The display may include a touch panel, and may receive a variety of information input through the touch panel and transmit the received information to the processor.

An image sensor 720 may be mounted in the main body 710. When a user's finger approaches the sensor 730 to measure a pulse wave signal, the image sensor 720 may capture an image of the finger and may transmit the captured image to the processor. In this case, based on the image of the finger, the processor may identify a relative position of the finger with respect to an actual position of the sensor 730, and may provide the relative position of the finger to the user through the display, so as to guide measurement of pulse wave signals with improved accuracy.

As described above, the processor may estimate bio-information based on the bio-signal measured by the sensor 730. In this case, as described above, the processor may adaptively obtain a progressive wave component, which is associated with the TPR-related feature, based on a variation in the CO-related feature, thereby improving accuracy in estimating bio-information.

In an example embodiment, a method for estimating bio-information may be implemented as computer-readable code written on a computer-readable recording medium. The computer-readable recording medium may be any type of recording device in which data is stored in a computer-readable manner.

Examples of the computer-readable recording medium include a ROM, a RAM, a CD-ROM, a magnetic tape, a floppy disc, an optical data storage, and a carrier wave (e.g., data transmission through the Internet). The computer-readable recording medium can be distributed over a plurality of computer systems connected to a network so that a computer-readable code is written thereto and executed therefrom in a decentralized manner. Functional programs, codes, and code segments needed for realizing the present invention can be easily deduced by programmers of ordinary skill in the art, to which the present invention pertains.

While example embodiments have been described, it will be understood by those skilled in the art that various changes and modifications can be made without changing technical ideas and features of the present disclosure. Thus, it is clear that the above-described embodiments are illustrative in all aspects and are not intended to limit the present disclosure. The invention is defined by the appended claims.

**Claims**

1. An apparatus for estimating bio-information, the apparatus comprising:

    a sensor (110, 620, 730) configured to obtain a bio-signal of a user; and
    a processor (120) configured to:

        obtain a heart rate from the obtained bio-signal;
        detect a heart rate variation compared to a reference heart rate measured in a resting state of the user at a reference time;
        estimate a variation in a cardiac output, CO,-related feature based on the heart rate variation;
        obtain a progressive wave component, which is associated with a total peripheral resistance, TPR,-related feature, from different positions of the bio-signal according to whether the variation in the CO-related feature is large or small, wherein the variation in the CO-related feature is estimated to be large, if the heart rate variation exceeds a predetermined threshold value, and the variation in the CO-related feature is estimated to be small, if the heart rate variation is less than or equal to the predetermined threshold value,
        wherein the processor (120) is further configured to:

            estimate the CO-related feature from the bio-signal,
            estimate the TPR-related feature based on the obtained progressive wave component, and
            estimate blood pressure by applying a predetermined blood pressure estimation model that defines a correlation between the CO-related feature, the TPR-related feature and blood pressure.

2. The apparatus of claim 1, wherein the sensor (110, 620, 730) comprises a pulse wave sensor, and the pulse wave sensor comprises a light source (731) configured to emit light onto the user, and a detector (732) configured to detect light reflected or scattered from the user.

3. The apparatus of claim 1, wherein the processor (120) is further configured to:

    obtain a heart rate from the bio-signal, and
    estimate the variation in the CO-related feature based on a variation in the heart rate compared to a reference heart rate obtained at a reference time.

4. The apparatus of claim 3, wherein the processor (120) is further configured to, in response to the variation in the heart rate exceeding a predetermined threshold value:

    estimate the variation in the CO-related feature, and
    obtain a progressive wave component based on the progressive wave component obtained at the reference time from the bio-signal.

5. The apparatus of claim 4, wherein the processor (120) is further configured to:

    obtain a second-order differential signal of the bio-signal, and
    obtain the progressive wave component based on a first local minimum point of the second-order differential signal.

6. The apparatus of claim 5, wherein the processor (120) is further configured to obtain, as the progressive wave component, one of a first amplitude of the bio-signal, which corresponds to a time of the first local minimum point, and a second amplitude of the bio-signal, which corresponds to an internal dividing point between the time of the first local minimum point and a time of a second local maximum point of the second-order differential signal.

7. The apparatus of claim 5, wherein the processor (120) is further configured to:

    detect an inflection point in a detection period of the second-order differential signal, and
    upon detecting the inflection point, obtain the progressive wave component based on the inflection point.

8. The apparatus of claim 7, wherein the detection period comprises a time interval between a first local maximum point and the first local minimum point of the second-order differential signal.

9. The apparatus of claim 4, wherein the processor (120) is further configured to, based on the variation in the heart rate being less than or equal to the predetermined threshold value, estimate the variation in the CO-related feature, and obtain a progressive wave component based on the progressive wave component obtained at the reference time from the bio-signal.

10. The apparatus of claim 9, wherein the processor (120) is further configured to obtain the progressive wave component based on a maximum amplitude point in a systolic phase of the bio-signal.

11. The apparatus of claim 10, wherein the processor (120) is further configured to obtain, as the progressive wave component, one of a first amplitude of the maximum amplitude point, and a second amplitude of the bio-signal which corresponds to an internal dividing point between a time of a first local minimum point of a second-order differential signal of the bio-signal and a time of the maximum amplitude point.

12. The apparatus of claim 1, wherein the processor (120) is further configured to estimate the variation in the CO-related feature based on a shape of a waveform of a reference bio-signal obtained at the reference time.

13. A computer-implemented method of estimating bio-information, the method comprising

obtaining a bio-signal of a user;
obtaining a heart rate from the obtained bio-signal;
detecting a heart rate variation compared to a reference heart rate measured in a resting state of the user at a reference time;
estimating (520) a variation in a cardiac output, CO,-related feature based on the heart rate variation;
obtaining (560) a progressive wave component, which is associated with a total peripheral resistance, TPR,-related feature, from different positions of the bio-signal according to whether the variation in the CO-related feature is large or small, wherein the variation in the CO-related feature is estimated to be large, if the heart rate variation exceeds a predetermined threshold value, and the variation in the CO-related feature is estimated to be small, if the heart rate variation is less than or equal to the predetermined threshold value;
estimating the CO-related feature from the bio-signal;
estimating the TPR-related feature based on the obtained progressive wave component; and
estimating blood pressure by applying a predetermined blood pressure estimation model that defines a correlation between the CO-related feature, the TPR-related feature and blood pressure.

14. The method of claim 13 configured to operate the apparatus for estimating the bio-information of one of claims 1 to 12.

**Patentansprüche**

1. Vorrichtung zum Schätzen von Bio-Informationen, wobei die Vorrichtung umfasst:

einen Sensor (110, 620, 730), konfiguriert zum Erhalten eines Biosignals eines Benutzers; und einen Prozessor (120), konfiguriert zum:

Erhalten einer Herzfrequenz aus dem erhaltenen Biosignal;
Detektieren einer Herzfrequenzvariation im Vergleich zu einer Referenzherzfrequenz, die in einem Ruhezustand des Benutzers zu einer Referenzzeit gemessen wurde;
Schätzen einer Variation in einem kardialen Ausstoß, (cardiac output) CO,-bezogenes Merkmal, basierend auf der Herzfrequenzvariation;
Erhalten einer progressiven Wellenkomponente, die mit einem Merkmal des gesamten peripheren Widerstands, (total peripheral resistance) TPR, verknüpft ist, aus verschiedenen Positionen des Biosignals, entsprechend dem, ob die Variation in dem CO-bezogenen Merkmal groß oder klein ist, wobei die Variation in dem CO-bezogenen Merkmal als groß geschätzt wird, wenn die Herzfrequenzvariation einen vorbestimmten Schwellenwert überschreitet, und die Variation in dem CO-bezogenen Merkmal als klein geschätzt wird, wenn die Herzfrequenzvariation kleiner oder gleich dem vorbestimmten Schwellenwert ist,
wobei der Prozessor (120) ferner konfiguriert ist, zum:

Schätzen des CO-bezogenen Merkmals aus dem Biosignal,
Schätzen des TPR-bezogenen Merkmals basierend auf der erhaltenen progressiven Wellenkomponente, und
Schätzen des Blutdrucks durch Anwenden eines vorbestimmten Blutdruckschätzmodells, das eine Korrelation zwischen dem CO-bezogenen Merkmal, dem TPR-bezogenen Merkmal und dem Blutdruck definiert.

2. Vorrichtung nach Anspruch 1, wobei der Sensor (110, 620, 730) einen Pulswellensensor umfasst, und
der Pulswellensensor eine Lichtquelle (731) umfasst, konfiguriert zum Emittieren von Licht auf den Benutzer, und einen Detektor (732), konfiguriert zum Detektieren von Licht, das von dem Benutzer reflektiert oder gestreut wird.

3. Vorrichtung nach Anspruch 1, wobei der Prozessor (120) weiterhin konfiguriert ist, zum:

Erhalten einer Herzfrequenz aus dem Biosignal, und
Schätzen der Variation des CO-bezogenen Merkmals basierend auf einer Variation der Herzfrequenz im Vergleich zu einer Referenzherzfrequenz, die zu einer Referenzzeit erhalten wurde.

4. Vorrichtung nach Anspruch 3, wobei der Prozessor (120) ferner konfiguriert ist, um in Reaktion auf das Überschreiten eines vorbestimmten Schwellenwerts durch die Variation der Herzfrequenz:

Schätzen der Variation des CO-bezogenen Merkmals, und
Erhalten einer progressiven Wellenkomponente basierend auf der progressiven Wellenkomponente, die zur Referenzzeit aus dem Biosignal erhalten wurde.

5. Vorrichtung nach Anspruch 4, wobei der Prozessor (120) ferner konfiguriert ist, zum:

Erhalten eines Differentialsignals zweiter Ordnung des Biosignals, und
Erhalten der progressiven Wellenkomponente basierend auf einem ersten lokalen Minimalpunkt des Differentialsignals zweiter Ordnung.

6. Vorrichtung nach Anspruch 5, wobei der Prozessor (120) ferner konfiguriert ist, zum Erhalten, als die progressive Wellenkomponente, einer ersten Amplitude des Biosignals, die einer Zeit des ersten lokalen Minimalpunkts entspricht, und einer zweiten Amplitude des Biosignals, die einem internen Teilungspunkt zwischen der Zeit des ersten lokalen Minimalpunkts und einer Zeit eines zweiten lokalen Maximalpunkts des Differenzsignals zweiter Ordnung entspricht.

7. Vorrichtung nach Anspruch 5, wobei der Prozessor (120) ferner konfiguriert ist, zum:

Detektieren eines Wendepunktes in einer Detektionsperiode des Differentialsignals zweiter Ordnung, und
Erhalten der progressiven Wellenkomponente basierend auf dem Wendepunkt nach dem Detektieren des Wendepunkts.

8. Vorrichtung nach Anspruch 7, wobei die Detektionsperiode ein Zeitintervall zwischen einem ersten lokalen Maximalpunkt und dem ersten lokalen Minimalpunkt des Differentialsignals zweiter Ordnung umfasst.

9. Vorrichtung nach Anspruch 4, wobei der Prozessor (120) ferner konfiguriert ist, zum Schätzen der Variation des CO-bezogenen Merkmals basierend auf der Variation der Herzfrequenz, die kleiner oder gleich dem vorbestimmten Schwellenwert ist, und zum Erhalten einer progressiven Wellenkomponente basierend auf der progressiven Wellenkomponente, die zur Referenzzeit aus dem Biosignal erhalten wurde.

10. Vorrichtung nach Anspruch 9, wobei der Prozessor (120) ferner konfiguriert ist, zum Erhalten der progressiven Wellenkomponente basierend auf einem Punkt maximaler Amplitude in einer systolischen Phase des Biosignals.

11. Vorrichtung nach Anspruch 10, wobei der Prozessor (120) ferner konfiguriert ist, zum Erhalten, als die progressive Wellenkomponente, einer von einer ersten Amplitude des maximalen Amplitudenpunktes und einer zweiten Amplitude des Biosignals, die einem internen Teilungspunkt zwischen einer Zeit eines ersten lokalen Minimalpunktes eines Differentialsignals zweiter Ordnung des Biosignals und einer Zeit des maximalen Amplitudenpunktes entspricht.

12. Vorrichtung nach Anspruch 1, wobei der Prozessor (120) ferner konfiguriert ist, zum Schätzen der Variation des CO-bezogenen Merkmals basierend auf einer Gestalt einer Wellenform eines Referenzbiosignals, das zur Referenzzeit erhalten wurde.

13. Computer-implementiertes Verfahren zum Schätzen von Bio-Informationen, wobei das Verfahren umfasst:

Erhalten eines Biosignals eines Benutzers;
Erhalten einer Herzfrequenz aus dem erhaltenen Biosignal;
Detektieren einer Herzfrequenzvariation im Vergleich zu einer Referenzherzfrequenz, die in einem Ruhezustand des Benutzers zu einer Referenzzeit gemessen wurde;
Schätzen (520) einer Variation in einem kardialen Ausstoß, (cardiac output) CO,-bezogenes Merkmal, basierend auf der Herzfrequenzvariation;
Erhalten (560) einer progressiven Wellenkomponente, die mit einem Merkmal des gesamten peripheren Widerstands, (total peripheral resistance) TPR, verknüpft ist, aus verschiedenen Positionen des Biosignals, entsprechend dem, ob die Variation in dem CO-bezogenen Merkmal groß oder klein ist, wobei die Variation in dem CO-bezogenen Merkmal als groß geschätzt wird, wenn die Herzfrequenzvariation einen vorbestimmten Schwellenwert überschreitet, und die Variation in dem CO-bezogenen Merkmal als klein geschätzt wird, wenn die Herzfrequenzvariation kleiner oder gleich dem vorbestimmten Schwellenwert ist;

Schätzen des CO-bezogenen Merkmals aus dem Biosignal;

Schätzen des TPR-bezogenen Merkmals basierend auf der erhaltenen progressiven Wellenkomponente; und

Schätzen des Blutdrucks durch Anwenden eines vorbestimmten Blutdruckschätzmodells, das eine Korrelation zwischen dem CO-bezogenen Merkmal, dem TPR-bezogenen Merkmal und dem Blutdruck definiert.

14. Verfahren nach Anspruch 13, konfiguriert zum Betreiben der Vorrichtung zum Schätzen der Bio-Informationen nach einem der Ansprüche 1 bis 12.

**Revendications**

1. Appareil d'estimation d'informations biologiques, l'appareil comprenant :

un capteur (110, 620, 730) configuré pour obtenir un signal biologique d'un utilisateur ; et
un processeur (120) configuré pour :

obtenir une fréquence cardiaque à partir du signal biologique obtenu ;
détecter une variation de fréquence cardiaque par rapport à une fréquence cardiaque de référence mesurée dans un état de repos de l'utilisateur à un temps de référence ;
estimer une variation d'une caractéristique liée au débit cardiaque, CO, sur la base de la variation de fréquence cardiaque ;
obtenir une composante d'onde progressive, qui est associée à une caractéristique liée à la résistance périphérique totale, TPR, à partir de différentes positions du signal biologique selon que la variation de la caractéristique liée au CO est grande ou petite, dans lequel la variation de la caractéristique liée au CO est estimée comme étant grande, si la variation de fréquence cardiaque dépasse une valeur seuil prédéterminée, et la variation de la caractéristique liée au CO est estimée comme étant petite, si la variation de fréquence cardiaque est inférieure ou égale à la valeur seuil prédéterminée,
dans lequel le processeur (120) est en outre configuré pour :

estimer la caractéristique liée au CO à partir du signal biologique,
estimer la caractéristique liée à la TPR sur la base de la composante d'onde progressive obtenue, et
estimer la pression artérielle en appliquant un modèle d'estimation de pression artérielle prédéterminé qui définit une corrélation entre la caractéristique liée au CO, la caractéristique liée à la TPR et la pression artérielle.

2. Appareil de la revendication 1, dans lequel le capteur (110, 620, 730) comprend un capteur d'onde de pouls, et

le capteur d'onde de pouls comprend une source lumineuse (731) configurée pour émettre de la lumière sur l'utilisateur, et un détecteur (732) configuré pour détecter la lumière réfléchie ou diffusée par l'utilisateur.

3. Appareil de la revendication 1, dans lequel le processeur (120) est en outre configuré pour :

obtenir une fréquence cardiaque à partir du signal biologique, et
estimer la variation de la caractéristique liée au CO sur la base d'une variation de la fréquence cardiaque par rapport à une fréquence cardiaque de référence obtenue à un temps de référence.

4. Appareil de la revendication 3, dans lequel le processeur (120) est en outre configuré pour, en réponse au fait que la variation de la fréquence cardiaque dépasse une valeur seuil prédéterminée :

estimer la variation de la caractéristique liée au CO, et
obtenir une composante d'onde progressive sur la base de la composante d'onde progressive obtenue au temps de référence à partir du signal biologique.

5. Appareil de la revendication 4, dans lequel le processeur (120) est en outre configuré pour :

obtenir un signal différentiel de second ordre du signal biologique, et
obtenir la composante d'onde progressive sur la base d'un premier point minimal local du signal différentiel de second ordre.

6. Appareil de la revendication 5, dans lequel le processeur (120) est en outre configuré pour obtenir, comme composante d'onde progressive, l'une d'une première amplitude du signal biologique, qui correspond à un temps du premier point minimal local, et d'une seconde amplitude du signal biologique, qui correspond à un point de division interne entre le temps du premier point minimal local et un temps d'un second point maximal local du signal différentiel de second ordre.

**7.** Appareil de la revendication 5, dans lequel le processeur (120) est en outre configuré pour :

détecter un point d'inflexion dans une période de détection du signal différentiel de second ordre, et

lors de la détection du point d'inflexion, obtenir la composante d'onde progressive sur la base du point d'inflexion.

**8.** Appareil de la revendication 7, dans lequel la période de détection comprend un intervalle de temps entre un premier point maximal local et le premier point minimal local du signal différentiel de second ordre.

**9.** Appareil de la revendication 4, dans lequel le processeur (120) est en outre configuré pour, sur la base du fait que la variation de la fréquence cardiaque est inférieure ou égale à la valeur seuil prédéterminée, estimer la variation de la caractéristique liée au CO, et obtenir une composante d'onde progressive sur la base de la composante d'onde progressive obtenue au temps de référence à partir du signal biologique.

**10.** Appareil de la revendication 9, dans lequel le processeur (120) est en outre configuré pour obtenir la composante d'onde progressive sur la base d'un point d'amplitude maximal dans une phase systolique du signal biologique.

**11.** Appareil de la revendication 10, dans lequel le processeur (120) est en outre configuré pour obtenir, comme composante d'onde progressive, l'une d'une première amplitude du point d'amplitude maximal et d'une seconde amplitude du signal biologique qui correspond à un point de division interne entre un temps d'un premier point minimal local d'un signal différentiel de second ordre du signal biologique et un temps du point d'amplitude maximal.

**12.** Appareil de la revendication 1, dans lequel le processeur (120) est en outre configuré pour estimer la variation de la caractéristique liée au CO sur la base d'une forme d'onde d'un signal biologique de référence obtenu au temps de référence.

**13.** Procédé mis en œuvre par ordinateur pour estimer des informations biologiques, le procédé comprenant :

l'obtention d'un signal biologique d'un utilisateur ;

l'obtention d'une fréquence cardiaque à partir du signal biologique obtenu ;

la détection d'une variation de fréquence cardiaque par rapport à une fréquence cardiaque de référence mesurée dans un état de repos de l'utilisateur à un temps de référence ;

l'estimation (520) d'une variation d'une caractéristique liée au débit cardiaque, CO, sur la base de la variation de fréquence cardiaque ;

l'obtention (560) d'une composante d'onde progressive, qui est associée à une caractéristique liée à la résistance périphérique totale, TPR, à partir de différentes positions du signal biologique selon que la variation de la caractéristique liée au CO est grande ou petite, dans lequel la variation de la caractéristique liée au CO est estimée comme étant grande, si la variation de fréquence cardiaque dépasse une valeur seuil prédéterminée, et la variation de la caractéristique liée au CO est estimée comme étant petite, si la variation de fréquence cardiaque est inférieure ou égale à la valeur seuil prédéterminée ;

l'estimation de la caractéristique liée au CO à partir du signal biologique ;

l'estimation de la caractéristique liée à la TPR sur la base de la composante d'onde progressive obtenue ; et

l'estimation de la pression artérielle en appliquant un modèle d'estimation de pression artérielle prédéterminé qui définit une corrélation entre la caractéristique liée au CO, la caractéristique liée à la TPR et la pression artérielle.

**14.** Procédé de la revendication 13 configuré pour faire fonctionner l'appareil d'estimation des informations biologiques de l'une des revendications 1 à 12.

# FIG. 1

FIG. 2

# FIG. 3

```
                                                        ┌─300
  ┌─────────────────────────────────────────────────────────┐
  │        ┌─310              ┌─320              ┌─330        │
  │  ┌──────────────┐  ┌──────────────┐  ┌──────────────┐    │
BIO- ─┼─▶│ VARIABILITY  │─▶│   FEATURE    │─▶│     BIO-     │    │
SIGNAL │  │  ESTIMATOR   │  │   OBTAINER   │  │ INFORMATION  │    │
  │  │              │  │              │  │  ESTIMATOR   │    │
  │  └──────────────┘  └──────────────┘  └──────────────┘    │
  └─────────────────────────────────────────────────────────┘
```

# FIG. 4A

FIG. 4B

# FIG. 4C

FIG. 4D

# FIG. 4E

# FIG. 4F

AMPLITUDE
PPG SIGNAL

$P_{sys}$ $P_{max}$

AMPLITUDE $T1$ $T_{max}$ TIME

SECOND-ORDER
DIFFERENTIAL
SIGNAL

0

TIME

# FIG. 5

START

MEASURE BIO-SIGNAL ⌐510

ESTIMATE VARIATION IN CO-RELATED FEATURE ⌐520

IS VARIATION LARGE? ⌐530

YES  NO

OBTAIN PROGRESSIVE WAVE COMPONENT HAVING RELATIVELY LARGE VARIATION ⌐540

OBTAIN PROGRESSIVE WAVE COMPONENT HAVING RELATIVELY SMALL VARIATION ⌐550

OBTAIN TPR-RELATED FEATURE BASED ON PROGRESSIVE WAVE COMPONENT ⌐560

ESTIMATE BIO-INFORMATION BASED ON CO-RELATED FEATURE AND TPR-RELATED FEATURE ⌐570

END

# FIG. 6

# FIG. 7

REFERENCES CITED IN THE DESCRIPTION

**Patent documents cited in the description**

- US 20200054223 A1
- US 2019110757 A1